# EUROPÄISCHE PATENTSCHRIFT

(11) **EP 0 671 006 B1**
(45) Veröffentlichungstag und Bekanntmachung des Hinweises auf die Patenterteilung: **05.02.1997**
(21) Anmeldenummer: 94900726.4
(22) Anmeldetag: 26.11.1993
(51) Int. Cl.: G01N 33/74

(54) **IMMUNOLOGISCHER SCHNELLTEST ZUR OPTISCHEN BESTIMMUNG VON PROGESTERON IN FLÜSSIGKEITEN**
IMMUNOLOGIC RAPID TEST FOR THE OPTICAL DETERMINATION OF PROGESTERONE IN FLUIDS
TEST IMMUNOLOGIQUE RAPIDE POUR LA DETECTION OPTIQUE DE LA PROGESTERONE DANS DES LIQUIDES

(30) Priorität: 26.11.1992 DE 9216110 U
(43) Veröffentlichungstag der Anmeldung: 13.09.1995
(73) Patentinhaber: BIOLAB GMBH, D-80995 München (DE)
(72) Erfinder: ARNSTADT, Klaus-Ingo, D-80995 München (DE)
(74) Vertreter: Fürniss, Peter, Dr.
(86) Internationale Anmeldenummer: DE9301126
(87) Internationale Veröffentlichungsnummer: WO9412883

(56) Entgegenhaltungen:
- EP-A- 0 223 349
- WO-A-89/02076
- DE-U- 9 216 110
- DATABASE WPI Week 8802, Derwent Publications Ltd., London, GB; AN 88-009835 & JP,A,62 272 156 (TEIKOKU HORMONE MFG LTD.) 26. November 1987
- CLINICAL CHEMISTRY Bd. 32, Nr. 5 , 1986 Seiten 763 - 767 J. DE BOEVER ET AL. 'Dirsct Solid-Phase Chemiluminescence Immunoassay for Salivary Progesterone.'

## Beschreibung

Die Erfindung betrifft ein Verfahren zur qualitativen/semiquantitativen Bestimmung von Progesteron in einer Flüssigkeit nach einem ELISA-Prinzip gemäß Patentanspruch 1. Die Erfindung betrifft weiterhin eine Testpackung zur Durchführung des genannten Verfahrens.

Die Bestimmung des Gelbkörperhormons Progesteron hat in der Tiermedizin und Tierzucht einen hohen Stellenwert bei der Kontrolle der Fruchtbarkeit, wie z.B. Prüfung von Zyklusstatus, Besamungszeitpunkt und Frühträchtigkeit. Mittels Progesteronbestimmung kann die An- oder Abwesenheit einer Gelbkörperfunktion ermittelt und dadurch Aussagen über das Vorhandensein von Ovarzyklus, Östrus (= Brunst = Befruchtungschance), Trächtigkeit oder einer Fertilitätsstörung bei verschiedenen Säugetierspezies gemacht werden, wie beispielsweise bei Rind, Pferd, Ziege, Schaf, Schwein, Hund, Kaninchen, Büffel, Kamel.

Seit der Entwicklung der ersten einsetzbaren Enzym-Immuntests für Progesteron wurae versucht, ihre Handhabung zu vereinfachen und die Laufzeit zu verkürzen mit dem Ziel, daß der jeweilige Progesterontest als sogennanter "Stalltest" oder "Stallgassentest" bereits vom Tierhalter auf dem Bauernhof durchgeführt werden kann. Hierzu wurden folgende Testkits entwickelt und auf den Markt gebracht bzw. vorgestellt:

**Tabelle 1**

| Testkit | Hersteller | Vertrieb für die BRD |
|---|---|---|
| Ovucheck^{R}-Praxistest | Cambridge Vet.Science,GB | Smith Kline GmbH |
| Progrestassay^{R} Milchprogesterontest | Pitman-Moore, USA | Janssen GmbH |
| Reprostrip-Progesteron-Schnelltest | Nochtech, Irland | A. Albrecht GmbH & Co, KG |
| Enzygnost^{R}-Milchprogesteron | Hoechst IQ (Bio) UK | Hoechst Veterinär GmbH für Photometer |

Auch wenn verschiedene Testverfahren die in sie gesetzten Erwartungen nicht erfüllen konnten, veranschaulichen sie jedoch, daß es für Progesteronbestimmungen mit der genannten Zielsetzung einen großen Bedarf gibt. Dies wird durch neuere Röhrchen-Tests nach dem ELISA-Prinzip aus den USA (HYGIA^{R} RPT Progesteron Test, Fa. Hartmann bzw. ImmuCell^{R}) und Großbritannien (CowsidE^{R}, Rapid TUBE Kit) unterstrichen, die zwar partielle, aber keine prinzipiellen Verbesserungen gegenüber den oben genannten Verfahren brachten.

Der Erfinder der vorliegenden Erfindung hat jedoch festgestellt, daß diese bekannten Verfahren mit einem in der Praxis entscheidenden Nachteil behaftet sind. Bei den bekannten Verfahren lassen sich zwar große Unterschiede des Progesterongehalts optisch leicht unterscheiden, wie sie tierphysiologisch z.B. bei Östrus (Brunst) im Vergleich zu Zyklusmitte und Trächtigkeit vorkommen. Unterschiede im unteren und mittleren Progesteronkonzentrationsbereich, als "Zwischenbereich" bezeichnet, sind jedoch schwer oder gar nicht zu erkennen. Durch diese eingeschränkte Aussagefähigkeit der bekannten Progesteron-Schnelltests ist deren Anwendung gerade in jenen Fällen mit einem hohen Risiko der Fehlaussage behaftet, wo ein Progesterontest besonders benötigt wird, nämlich bei Tieren mit nicht eindeutigem Brunstverhalten, z.B. "stille Brunst", "Brunstnähe", "Mittzyklusfollikel" oder mit anluteinisierten Zysten (vgl. Arnstadt, K.-I. u. A. Sobiraj, top agrar 2/87 (1987) R24-R26).

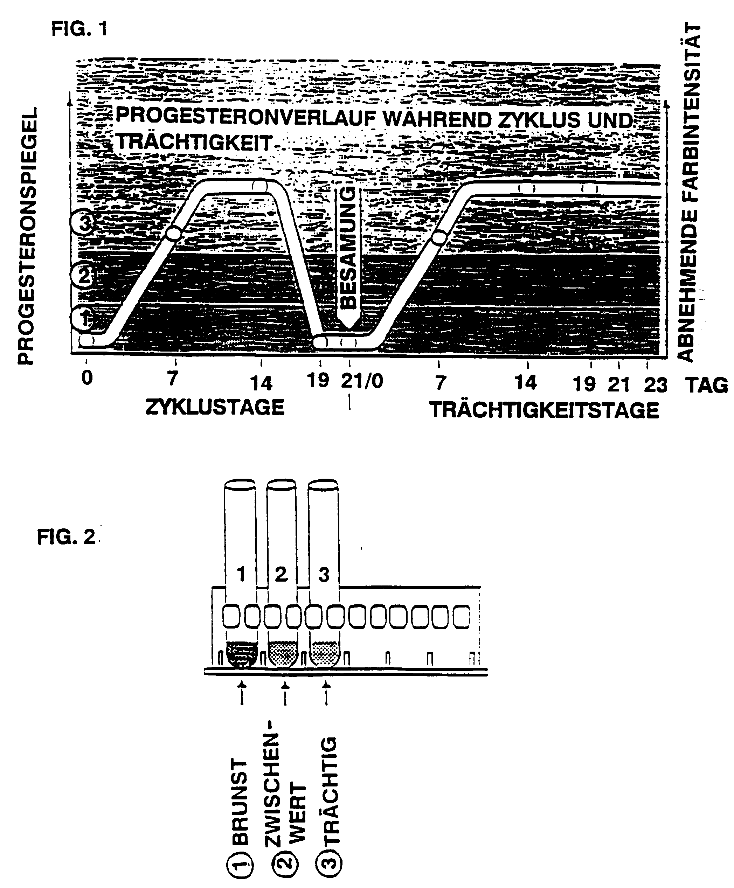

So werden z.B. Kühe im Progesteronzwischenbereich von ca. 2 - 6 ng/ml mit den bisherigen, bekannten Schnelltests für Milchproben oftmals fälschlicherweise dem Status abwesender Corpus luteum-Funktion zugeordnet (falsch negatives Ergebnis). Bei Kühen bedeutet das "Hinweis auf Vorliegen einer Brunst", die in Wirklichkeit bereits beendet ist oder andererseits erst in einigen Tagen einsetzt. Das kann eine falsche Terminierung des Östrus und der Besamung zur Folge haben oder eine ungeeignete Therapie des Tieres bei dadurch fehldiagnostizierten Fruchtbarkeitsstörungen provozieren (vgl. Fig. 1)

Falsch positive Ergebnisse, die ebenfalls mit bisherigen Testverfahren unzureichender Genauigkeit erzielt werden, können eine erfolgreiche Befruchtung vortäuschen. Die Konsequenzen, die ein Tierzüchter oder Tierarzt aus einer solchen Fehlinformaticn ziehen kann, sind bedenklich und oft mit einem wirtschaftlichen Nachteil für den Tierhalter verbunden.

Der Erfindung liegt daher die Aufgabe zugrunde, bei der Progesteronbestimmung mittels Schnelltest auch den "Zwischenbereich", d. h. den Bereich niedriger, aber vorhandener Progesteronkonzentration, deutlich anzuzeigen und so eine gültigere Aussage über das Reproduktionsgeschehen zu erhalten, als bisher möglich war.

Diese Aufgabe wird erfindungsgemäß durch ein Verfahren nach dem ELISA-Prinzip zur qualitativ-semiquantitativen Bestimmung von Progesteron in einer Flüssigkeit gelöst, bei dem man
a) einige Tropfen der zu untersuchenden Flüssigkeit nimmt,
b) diese mit einer mit einem Antikörper beschichteten Oberfläche in Berührung bringt,
c) die Flüssigkeit mit einer wäßrigen ProDenverdünneriösung versetzt,
d) eine Progesteron-Enzymkonjugat-Lösung hinzugibt, die vorzugsweise mit den noch freien Bindungsstellen der fixierten Antikörper reagiert,
e) die Mischung der Lösungen, d. h die nicht vom Antikörper gebundenen Substanzen, entfernt,
f) das gebundene Enzym durch eine Farbreaktion nachweist und anhand der Intensität der entstehenden Farbe den Progesterongehalt der zu untersuchenden Flüssigkeit bestimmt.

Die Schritte b) und c) können auch in umgekehrter Reihenfolge oder gleichzeitig durchgeführt werden.

Gemäß einer bevorzugten Ausführungsform wird der Progesterongehalt in Milch und Blut bestimmt.

Bei einer anderen Ausführungsform wird eine Membran eingesetzt. Hier läuft die Reaktion auf einer mit Antikörpern präparierten Filtermembran eines Trichtersystems ab, wobei die zu untersuchenden Flüssigkeiten (Milch, BLutplasma oder -serum und Reagenzien) kontinuierlich entfernt werden.

Gemäß einer weiteren Ausführungsform enthält die Probenverdünner-Lösung ein oder mehrere Puffersalze. Besonders zuverlässig kann die Progesteronbestimmung durchgeführt werden, wenn die Probenverdünner-Lösung weiterhin einen Emulgator enthält. Als Emulgator können vorteilhafterweise ein Detergens oder ein anderer (Steroid-)Hormon bzw. Lipid vermittelnder Zusatzstoff, wie z.B. Tween 20^{R}, ein Protein, Lipoprotein, Immunglobulin oder auch Gelatine eingesetzt werden. Der Zusatz solcher Emulgatoren zur ProbenverdünnerLösung bewirkt unter anderem, daß das lipophile Progesteron in der zu untersuchenden Flüssigkeit leichter und zuverlässiger mit dem auf der Oberfläche haftenden Antikörpern reagiert.

Wird erfindungsgemäß im Progesteron-Enzym-Konjugat Peroxidase als Enzym eingesetzt, so kann man bekannte Substratreaktionen einsetzen, wie z. B. die mit 3,3',5,5'-Tetramethylbenzidin (TMB), einsetzen, die eine Blaufärbung verursacht.

Vorteilhafterweise führt man den optischen Nachweis des Enzyms im Fall einer Peroxidase (HRP) mit einer Substratpufferlösung durch, die bereits ein Substrat, z. B. H₂O₂ enthält und dem das farbstoffbildende zweite Substrat in einer raschen Startreaktion, also in einem zweiten Schritt, zugegeben wird.

Gemäß einer bevorzugten Ausführungsform kann das erfindungsgemäße Verfahren mit einem ELISA-Teströhrchen durchgeführt werden. Dies nat den Vorteil, daß die Reaktionsgefäße als Träger der mit Antikörper zu beschichtenden Oberfläche eine handliche Größe besitzen.

Das mit der erfindungsgemäßen Testpackung durchführbare Verfahren gibt schnell und zuverlässig qualitative und semiquantitative Aussagen über Proöstrus, Östrus, Zyklusmitte, Trächtigkeit und insbesondere über den bisher mit optischen bzw. visuellen Schnelltests kaum erfaßbaren Zwischenbereich der Östrusnähe bei schwachem Progesterongehalt. Dieser wird dadurch als solcher erkennbar und liefert die wichtige Aussage: Das Tier ist weder tragend noch befindet es sich im Östrus.

Erfindungsgemäß wird der Östrusbereich gegenüber bekannten Testpackungen besser vom Zwischenbereich oder umgekehrt abgegrenzt und die Fehlbeurteilung "gelbkörperinaktiv" im Fall schwacher, aber immerhin vorhandener Corpus luteum-Aktivität am Ende des Zyklus vor einem Östrus oder am Zyklusbeginn nach einer bereits stattgefundenen Ovulation in hohem Maße vermieden.

Weitere Vorteile sind die geringe Abhängigkeit des vorgeschlagenen Schnelltests vom Milchfettgehalt, sofern er mit Milch durchgeführt wird, oder vom Lipidgehalt, wenn er mit lipämischen Seren ausgeführt werden muß.

Ein weiterer Vorteil gegenüber den bekannten Tests ist eine große Reproduzierbarkeit seiner Ergebnisse.

Durch Vergleich der im Test erhaltenen Farbe des bzw. der Probenröhrchen mit Vergleichsröhrchen, die mit der Standardprobe "Östrus" sowie mit der Kontrollprobe "Trächtig" durchgeführt wurden, iassen die Ergebnisse eine eindeutige Beurteilung zu.

Gemäß einer bevorzugten Ausführungsform enthält die erfindungsgemäße Testpackung daher zusätzlich je einen Behälter für
e) eine Standardprobe "ÖSTRUS" (=progesteronfreier Standard) und
f) eine Kontrollprobe "Corpus luteum aktiv" oder "TRÄCHTIG" (progesteronhaltige Kontrolle) für die Progesteron-dominante Phase des Fruchtbarkeitsgeschehens.

Basis (Matrix) der Standard- und Kontrollproben sind je nach Testanwendung Milch, Blut (Plasma oder Serum) oder Speichel, denen ein Konservierungsmittel, wie z.B. Kaliumbichromat oder Natriumazid zugesetzt wird.

Gemäß einer weiteren Ausführungsform enthält die Testpackung in bekannter Weise eine Anzahl von Einmalpipetten und ein Röhrchengestell, wodurch sämtliche Hilfsmittel zur Durchführung des Tests griffbereit vorhanden sind.

Als mit einem Antikörper beschichtete Oberfläche dient vorzugsweise die Innenwand von Teströhrchen. Statt einem Teströhrchen können auch andere Reaktionsgefäße eingesetzt werden. Für diesen Zweck kann man, z. B. auch die Innenwand der Kavitäten (Vertiefungen) von Mikrotiterplatten oder Mikrotiterstreifen oder die Oberfläche eines Stäbchens oder Streifens aus Kunststoff oder Papier verwenden. Vorteilhaft läßt sich hierfür auch die permeable Membran eines Trichter-, Durchlauf- bzw. Absaugsystems verwenden.

Die Probenverdünnerlösung b) enthält z.B. die folgenden Substanzen:Puffersalze in einer Konzentration von 0,01 bis 0,3 molar, die einen pH-Bereich von 5,0 bis 8,5 gewährleisten; einen oder mehrere Emulgator(en) z.B. ein Detergens, wie Tween 20^{R}, Gelatine oder andere für sich allein oder in Kombination miteinander im Konzentrationsbereich von 0,02 bis 5 Gew.-%.

Die Progesteron-Enzym-Konjugat-Lösung c) ist an sich bekannt und enthält z.B. 0,01 bis 0,5 ng/ml Progesteron-Peroxydase-Konjugat.

Das Substrat d) kann z.B. eine Substratpuffer-Lösung und eine Chromogen-Lösung enthalten. Die Substratpuffer-Lösung entspricht dem Stand der Technik. Sie enthält z.B. 0,1 molaren Citratpuffer, pH 4,7 und 0,01 bis 0,20 % Mol Wasserstoffperoxyd. Die Chromogen-Lösung ist ebenfalls bekannt und enthält vorzugsweise TMB (3,3',5,5'-Tetramethylbenzidinhydrochlorid) in verdünnter Salzsäure oder in Citronensäurepuffer.

In den Zeichnungen gibt
- Fig. 1: den Progesteronverlauf bei der Kuh während Zyklus und Trächtigkeit an, während
- Fig. 2: das Ergebnis des vorgeschlagenen Schnelltests verdeutlicht.

Bei der Durchführung des Schnelltests mit der erfindungsgemäßen Testpackung wird eine (z.B. mittels Tropfpipette) abgemessene Menge der Milch-, Blut- oder Speichelprobe mit einer definierten Menge Pufferlösung ("Prooenverdünner") im Teströhrcnen verdünnt. Während dieser Vorinkubation emulgieren Lipidbestandteile der Probe, wodurch das zu bestimmende Progesteron leichter zugänglich wird und die Immunogen- (bzw. Antigen-) Antikörperreaktion bereits startet. Erst in einem zweiten Reaktionsschritt reagiert das der Testmischung hinzugetropfte Progesteron-Enzym-Konjugat mit den an die Röhrchenwand fixierten Antikörpern, vorzugsweise mit den noch nicht durch Progesteron besetzten Bindungsstellen. Nach dem Ausgießen der Testmischung und dem Auswaschen der Röhrchen, wodurch die Testmischung mit dem überschüssigen, ungebundenen Konjugat entfernt wird, erfolgt nach Zugabe von Substratlösung die Entwicklung einer Färbung, deren Intensität von der Menge des gebundenen Hormon- bzw. Hapten- (hier Progesteron-) Enzym-Konjugats direkt und vom Progesterongehalt der Probe umgekehrt proportional abhängt. Hierbei reagiert nach dem bekannten ELISA-Prinzip das über eine Antikörper-Hormon-Enzym-Brücke an die Röhrchenwand gebundene Enzym.

Die Beurteilung der Färbung und Interpretation der Meßergebnisse kann vorteilhafterweise anhand von definierten Referenzproben, der progesteronfreien Standardprobe (= Östrus) und der progesteronhaltigen Kontrollprobe (= Trächtig) erfolgen, die im jeweiligen Testansatz mitlaufen. Bei Bedarf kann aus diesen Referenzproben durch Mischen, z.B. im Verhältnis 2:1, eine weitere Referenzprobe "Zwischenwert" gewonnen werden, was die Sicherheit der Testinterpretation im Einzelfall erhöhen kann.

Bei Durchführung dieses Verfahrens im Labor, beispielsweise durch einen Tierarzt mit Laborausrüstung (Photometer), kann nach der visuellen Beurteilung die enzymatische Reaktion mit einer Stoplösung, z. B. verdünnter Schwefelsäure gestoppt und die Färbung dokumentiert werden, z. B. durch quantitative photometrische Messung. Hierdurch wird die Genauigkeit der Testbeurteilung noch erhöht. Für diesen Zweck kann die erfindungsgemäße Testpackung einen weiteren Behälter mit Stop-Lösung enthalten.

Der zusätzliche Aufwand des erfindungsgemäßen Schnelltests gegenüber bisherigen Röhren-Schnelltests durch Einführung eines Verdünnungsschrittes im Teströhrchen mit Vorinkubation verlängert zwar insgesamt die Testdauer bzw. die Inkubationszeiten um ca. 3-5 Minuten auf 10-15 Minuten. Dieser Nachteil gegenüber rein kompetitiven Röhrchen- und Mikrotiter-Schnelltests ist jedoch geringfügig, verglichen mit dem Vorteil der insgesamt zuverlässigeren Testgenauigkeit im allgemeinen und der mit Hilfe des erfindungsgemäßen Schnelltest differenzierbaren Testaussage "Zwischenbereich" im besonderen.

### Beispiele 1 - 3

### Milch-Progesteron

Der Test wird bei Raumtemperatur, vorzugsweise 18 - 25 °C, ausgeführt. Es werden eine oder mehrere Milchproben (Vollmilch oder Nachgemelk) genommen. Bei jedem Testlauf werden wegen der erforderlichen Referenzfärbung ein oder zwei Standards mitgemessen. Diese werden der Flasche Standardmilch "Brunst" und einer Kontrollmilch "Trächtig" entnommen. Eine zusätzliche Kontrolle "Zwischenbereich" wird bei Bedarf aus zwei Teilen Standard-"Östrus" und einem Teil Kontrollmilch "Trächtig" gemischt.

3 Tropfen Standard- oder Probenmilch (Beispiel 2: 5 Tropfen, Beispiel 3: 3 Tropfen) werden in den unteren Teil des jeweiligen Röhrchens gegeben. 5 Tropfen Probenverdünner (Beispiel 2: 3 Tropfen, Beispiel 3: 5 Tropfen) werden pro Röhrchen hinzugetropft. Der Röhrcheninhalt wird 10 Sekunden rasch gemischt und 3 Minuten (Beispiel 2 und 3: 5 Minuten) stehengelassen. Dann gibt man 3 Tropfen Progesteron-EnzymKonjugat (Beispiel 2: 3 Tropfen, Beispiel 3: 2 Tropfen) pro Röhrchen hinzu, mischt 10 Sekunden und läßt 3 Minuten stehen. Der Röhrcheninhalt wird ausgeleert und das Röhrchen 5 mal mit kaltem Wasser gespült. Anschließend werden 6 bis 10 Tropfen Substratpuffer bis zu einer bei allen Röhrchen eines Testdurcngangs gleichen Füllhöhe hinzugegeben. Durch Zugabe von 1 Tropfen Chromogen (Beispiel 2: 1 Tropfen, Beispiel 3: 2 Tropfen) wird die Enzymreaktion gestartet. Ab 3 - 5 Minuten wird die Färbung der Proben im Vergleich zu Standard und Kontrolle beurteilt. Es werden zur Auswertung unterschieden (vergl. Fig. 2):
1) Kräftige Farbe Progesterongehalt niedrig: Kann Brunst oder Brunstnähe bedeuten, wenn zusätzlich äußere Brunstsymptome festzustellen sind. Eindeutige Aussage: Kuh ist nicht trächtig.
2) Mittlere Färbung - Progesteronwert im Zwischenbereich Kann Brunstnähe bedeuten. In diesem Fall befindet sich die Kuh 2 bis 3 Tage vor oder 3 bis 4 Tage nach der Brunst. Eindeutige Aussage: Die Kuh ist weder in Brunst, noch ist sie trächtig.
3) Schwache Färbung - Progesterongehalt hoch Kann Trächtigkeit, z. B. 19 - 23 Tage nach Besamung bedeuten, oder die Kuh befindet sich im Zyklus. Eindeutige Aussage: Die Kuh ist nicht in Brunst.

### Beispiele 4 und 5

### Schnelltest für die Blutproben

Als Probenmaterial für die Blutprobe kann Plasma, Serum oder unzentrifugiertes Vollblut eingesetzt werden. Der Test wird bei Raumtemperatur, vorzugsweise 18 - 25 °C ausgeführt. Man gibt 5 Tropfen der Blutprobe (Serum, Plasma oder Vollblut) in jedes Röhrchen. Es werden 3 Tropfen Proben-Verdünner hinzugefügt. an schüttelt 10 Sekunden und läßt 3 - 5 Minuten stehen. Es werden 2 - 3 Tropfen Progesteron-Enzym-Konjugat zugegeben. Man schüttelt 10 Sekunden und läßt 3 Minuten stehen. Die Röhrchen werden ausgeleert und 5 mal mit kaltem Leitungswasser gewaschen und ausgeschleudert. (Unzentrifugiertes Blut: 7 bis 8 mal waschen.) 6 bis 10 Tropfen Substratpuffer werden in die Röhrchen gegeben. Dann wird die Reaktion zügig mit je 1 oder 2 Tropfen Chromogen gestartet. Ab ca. 3 - 5 Minuten wird die Färbung der Probe im Vergleich zu Standard und Kontrolle beurteilt. Die Auswertung folgt analog zu den Beispielen 1 - 3.

### Beispiele 6 und 7

### Schnelltest auf Mikrotiter-Platten und -streifen

Als Probenmaterial dienen Milch- (Beispiel 6) und Blutproben (Beispiel 7). Der Test wird analog der Beispiele 1 - 5 wie folgt ausgeführt. Man pipettiert 1 Tropfen Milch (Beispiel 7: 2 Tropfen Blut) pro Kavität, fügt 1 Tropfen Verdünner hinzu, mischt 10 Sekunden intensiv und inkubiert 5 Minuten bei Raumtemperatur. Nach Zugabe von 2 Tropfen Progesteron-Enzymkonjugat werden 3 - 5 Minuten inkubiert, die Kavitäten ausgewaschen und die gebundene Enzymkavität mit 4 Tropfen Substratpuffer und 1 Tropfen Chromogen nachgewiesen. Die Auswertung erfolgt analog zu den Beispielen 1 - 3.

## Patentansprüche

1. Schnelltest zur qualitativen/semiquantitativen Bestimmung von Progesteron in Milch (Vollmilch) oder Blut nach einem ELISA-Prinzip, wobei man
a) einige Tropfen der zu untersuchenden Flüssigkeit nimmt,
b) diese mit einer mit einem spezifischen Antikörper beschichteten Oberfläche in Berührung bringt,
c) die Flüssigkeit mit einer wäßrigen Probenverdünnerlösung versetzt,
d) eine Progesteron-Enzymkonjugat-Lösung hinzugibt, die vorzugsweise mit den noch freien Bindungsstellen der fixierten Antikörper reagiert,
e) die Mischung der Lösungen, d. h. die nicht vom Antikörper gebundenen Substanzen, entfernt,
f) das gebundene Enzym durch eine Farbreaktion nachweist und anhand der visuell beurteilten Intensität der entstehenden Farbe den Progesterongehalt der zu untersuchenden Flüssigkeit bestimmt,
wobei man die Schritte b) und c) auch in umgekehrter Reihenfolge oder gleichzeitig durchführen kann.

2. Schnelltest nach Anspruch 1, dadurch gekennzeichnet, daß die Probenverdünnerlösung ein oder mehrere Puffersalz(e) enthält.

3. Schnelltest nach Anspruch 2, dadurch gekennzeichnet, daß die Probenverdünnerlösung Emulgator enthält.

4. Schnelltest nach Anspruch 3, dadurch gekennzeichntet, daß man als Emulgator ein Detergens oder einen anderen hormonvermittelnden Zusatzstoff, wie z. B. Tween-20®, ein Lipoprotein, Immunglobulin, Protein, oder Gelatine einsetzt.

5. Schnelltest nach einem der Ansprüche 1 bis 4, dadurch gekennzeichnet, daß man als Progesteron-Enzym-Konjugat ein Progesteron-Peroxidase-Konjugat einsetzt.

6. Schnelltest nach Anspruch 5, dadurch gekennzeichnet, daß man zum optischen Nachweis des Enzyms eine Substratpufferlösung einsetzt, die bereits ein Substrat enthält und der als zweites Substrat eine Chromogenlösung hinzugefügt wird.

7. Schnelltest nach einem der Ansprüche 1 bis 6, dadurch gekennzeichnet, daß man als mit einem Antikörper beschichtete Oberfläche die Innenwand von Teströhrchen verwendet.

8. Testpackung für einen qualitativen/semiquantitativen Schnelltest auf Progesteron in einer Flüssigkeit nach dem ELISA-Prinzip, enthaltend
a) Gefäße mit einer mit anti-Progesteron-Antikörpern bzw. anti-Progesteron-IgG beschichteten Oberfläche, und mindestens je einen Behälter für
b) ProbenverdünnerLösung und
c) Progesteron-Enzymkonjugat-Lösung, sowie
d) mindestens ein Substrat oder eine Substratmischung zum optischen Nachweis des Enzyms durch die Farbreaktion.

9. Testpackung nach Anspruch 8, gekennzeichnet durch eine oder mehrere Standards bzw. Kontrollen mit bekanntem Gehalt der nachzuweisenden Verbindung, wie
e) eine progesteronfreie oder nahezu progesteronfreie Standardprobe;
f) eine progesteronhaltige Kontrollprobe

10. Testpackung nach Anspruch 8 und/oder 9, gekennzeichnet durch eine Stopplösung zur Beendigung der Farbreaktion und Fixierung der Farbe.

## Claims

1. A rapid test for qualitative/semiquantitative determination of progesterone in milk (whole milk) or blood on an ELISA principle, in which
a) a few drops of the fluid to be investigated are taken,
b) these are brought into contact with a surface coated with a specific antibody,
c) the fluid is mixed with an aqueous sample diluent solution,
d) a progesterone-enzyme conjugate solution is added which preferably reacts with the still-free binding sites of the immobilized antibodies,
e) the mixture of the solutions, i.e. the substances not bound by the antibody, are removed,
f) the bound enzyme is detected by a color reaction and using the visually evaluated intensity of the resulting color the progesterone content of the fluid to be investigated is determined,
it also being possible to carry out steps b) and c) in reverse order or simultaneously.

2. The rapid test as claimed in claim 1, wherein the sample diluent solution contains one or more buffer salt(s).

3. The rapid test as claimed in claim 2, wherein the sample diluent solution contains emulsifier.

4. The rapid test as claimed in claim 3, wherein the emulsifier employed is a detergent or another hormone-mediating additive, such as e.g. Tween-20®, a lipoprotein, immunoglobulin, protein or gelatin.

5. The rapid test as claimed in one of claims 1 to 4, wherein the progesterone-enzyme conjugate employed is a progesterone-peroxidase conjugate.

6. The rapid test as claimed in claim 5, wherein a substrate buffer solution is employed for the optical detection of the enzyme which already contains a substrate and to which a chromogen solution is added as a second substrate.

7. The rapid test as claimed in one of claims 1 to 6, wherein the surface coated with an antibody used is the inner wall of test tubes.

8. A test pack for a qualitative/semiquantitative rapid test for progesterone in a fluid on the ELISA principle, containing
a) vessels having a surface coated with anti-progesterone antibodies or anti-progesterone IgG, and at least one container each for
b) sample diluent solution and
c) progesterone-enzyme conjugate solution, and also
d) at least one substrate or a substrate mixture for the optical detection of the enzyme by means of the color reaction.

9. The test pack as claimed in claim 8, which comprises one or more standards or controls having a known content of the compound to be detected, such as
e) a progesterone-free or nearly progesterone-free standard sample;
f) a progesterone-containing control sample.

10. The test pack as claimed in claims 8 and/or 9, which comprises a stop solution for ending the color reaction and fixing the color.

## Revendications

1. Test rapide pour la détermination qualitative/semi-quantitative de progestérone dans le lait (lait entier) ou dans le sang conformément à un principe ELISA, dans lequel:
a) on prélève quelques gouttes du liquide à analyser,
b) on amène ces dernières en contact avec une surface enduite d'un anticorps spécifique,
c) on ajoute au liquide une solution aqueuse de dilution d'échantillon,
d) on y ajoute une solution de conjugués de progestérone-enzyme qui réagit de préférence avec les endroits de liaison encore libres de l'anticorps fixé,
e) on élimine le mélange des solutions, c'est-à-dire les substances qui n'ont pas été liées par l'anticorps,
f) on analyse l'enzyme liée à l'aide d'une réaction de coloration et sur base de l'intensité évaluée à l'oeil nu de la couleur obtenue; on détermine la teneur en progestérone du liquide à analyser,
dans lequel on peut également effectuer les étapes b) et c) dans l'ordre inverse ou simultanément.

2. Test rapide selon la revendication 1, caractérisé en ce que la solution de dilution d'échantillon contient un ou plusieurs sels tampons.

3. Test rapide selon la revendication 2, caractérisé en ce que la solution de dilution d'échantillon contient un émulsifiant.

4. Test rapide selon la revendication 3, caractérisé en ce qu'on met en oeuvre comme émulsifiant un détergent ou un autre additif procurant des hormones tel que par exemple Tween-20®, une lipoprotéine, une immunoglobuline, une protéine ou de la gélatine.

5. Test rapide selon l'une quelconque des revendications 1 à 4, caractérisé en ce que, comme conjugué de progestérone-enzyme, on met en oeuvre un conjugué de progestérone-peroxydase.

6. Test rapide selon la revendication 5, caractérisé en ce que pour l'analyse optique de l'enzyme, on met en oeuvre une solution tampon faisant office de substrat qui contient déjà un substrat et auquel on ajoute, comme second substrat, une solution chromogène.

7. Test rapide selon l'une quelconque des revendications 1 à 6, caractérisé en ce qu'on utilise comme surface enduite à l'aide d'un anticorps, la paroi interne de petits tubes à essais.

8. Nécessaire d'essai pour un test rapide qualitatif/semi-quantitatif pour détecter la présence de progestérone dans un liquide conformément au principe ELISA, contenant
a) des récipients dont la surface est enduite avec des anticorps anti-progestérone, respectivement avec une IgG anti-progestérone, et au moins respectivement un récipient pour
b) une solution de dilution d'échantillon et
c) une solution de conjugué de progestérone-enzyme, ainsi que
d) au moins un substrat ou un mélange de substrats pour l'analyse optique de l'enzyme par la réaction de coloration.

9. Nécessaire d'essai selon la revendication 8, caractérisé par une ou plusieurs normes, respectivement un ou plusieurs témoins avec une teneur connue du composé à analyser, tels que
e) un échantillon normal exempt de progestérone ou pratiquement exempt de progestérone;
f) un échantillon témoin contenant de la progestérone.

10. Nécessaire d'essai selon la revendication 8 et/ou 9, caractérisé par une solution d'arrêt pour mettre un terme à la réaction de coloration et pour fixer la couleur.
